# EUROPEAN PATENT APPLICATION

(11) **EP 4 053 559 A1**
(43) Date of publication of application: **07.09.2022**
(21) Application number: 21382173.9
(22) Date of filing: 01.03.2021
(51) Int. Cl.: G01N 33/68, A61K 38/48

(54) **METHODS FOR THE MEASUREMENT OF PROTEIN C AND ACTIVATED PROTEIN C**

(71) Applicant: Consorcio Centro de Investigación Biomédica en Red, M.P. (CIBER), 28029 Madrid (ES); Universitat de València, 46010 Valencia (ES); Instituto de Investigación Sanitaria INCLIVA, 46010 Valencia (ES)
(72) Inventor: GARCÍA GIMÉNEZ, José Luis, 46010 Valencia (ES); PALLARDÓ CALATAYUD, Federico Vicente, 46010 Valencia (ES); MENA MOLLÁ, Salvador, 46010 Valencia (ES); CARBONELL MONLEÓN, Nieves, 46010 Valencia (ES)
(74) Representative: Hoffmann Eitle

(57) **Abstract**

The present invention relates to a method for measuring the levels of Protein C (PROC), preferably Activated Protein C (APC), in a subject in need thereof, the method comprising a step of performing an enzymatic digestion of one or more biological samples isolated from the subject, and a step of measuring the levels of the peptide of SEQ ID NO 1 (LGEYDLR), wherein the levels of SEQ ID NO 1 corresponds to the levels of PROC, preferably APC, present in said one or more biological samples. Methods for classifying subjects and prognosticating the response to treatments are also included.

## Description

### TECHNICAL FIELD OF THE INVENTION

The present invention is related to the medical field, in particular to the field of measuring biomarkers by using mass spectrometry-based methods. More particularly, the present invention is related to mass spectrometry-based methods for the measurement/quantification of Protein C, preferably Activated Protein C, using specific peptides that correlate with those proteins.

### BACKGROUND OF THE INVENTION

In the Third International Consensus Definitions for Sepsis and Septic Shock (Sepsis-3), sepsis was defined as a life-threatening organ-dysfunction (total SOFA score ≥2 points assuming to be zero the baseline SOFA in patients not known to have preexisting organ dysfunction) that arises when the body's response to an infection damages its own tissues. Septic shock was defined as a subset of sepsis in which profound circulatory, cellular, and metabolic abnormalities are associated with increased risk of mortality than with sepsis alone. Yet despite the advances made in vaccines, antibiotics and acute care in intensive care units (ICUs), sepsis affects as many as 49 million people, remains the leading cause of death from infection with over 11 million deaths annually worldwide, and is the most frequent cause of death in ICUs.

Disseminated intravascular coagulation (DIC) is an acquired clinic biological syndrome characterized by widespread activation of coagulation leading to fibrin deposition in the vasculature, organ dysfunction, consumption of clotting factors and platelets, and life-threatening haemorrhage.

Protein C deficiency (PROC deficiency) is a rare genetic disorder characterized by a deficiency of protein C, so subjects affected by this rare disease are at risk for developing clots, particularly a type of blood clot called deep vein thrombosis. Although very rare, there is a severe form that is present at birth (congenital) and can potentially cause widespread small clots in the body and life-threatening complications in infancy. Patients with undetectable protein C levels usually manifest the disease several hours to days after birth, with purpura fulminans or massive venous thrombosis.

Protein C (PROC) has antithrombotic, anti-inflammatory and profibrinolytic properties which may play a role in limiting the injurious effects of the severe systemic response to septicemia. The role of PROC anticoagulant pathway in the pathogenesis of DIC has been studied in experimental models of sepsis-induced DIC and in patients with sepsis. Protein C deficiency is a frequent finding in patients with sepsis due mainly to enhanced consumption, impaired liver synthesis, and vascular leakage.

Activated protein C (APC) is a 56-kDa blood vitamin K-serine protease and results from cleavage of an Arg169-Leu170 peptide bond from the Protein C (PROC), releasing a dodecapeptide (residues 158-169) from the heavy chain. APC demonstrated anticoagulant, cytoprotective, anti-inflammatory and cell-signaling activities. Among the recent assigned roles for APC, it is widely known the role of APC as a principal regulator of thrombin generation, and its role controlling blood coagulation by proteolytic degradation of procoagulant activated cofactors essential for fibrin clot development. Furthermore, APC has been shown to contribute to the adaptive immune response in several preclinical models of autoimmune disease.

Several studies demonstrate the therapeutic uses of APC and thus treatments with recombinant human APC (rhAPC) arose in the market. The acquired APC deficiency in sepsis-induced DIC is related to hypercoagulable state and increased mortality in septic patients. It has been demonstrated that treatment with APC reduced 28-day mortality of patients with severe sepsis, including patients retrospectively assigned to a subgroup with sepsis-associated DIC. However, treatment with APC did not have any positive effects in other patient groups, suggesting that only septic patients with DIC would be benefit from the use of APC treatment (i.e. Xigris or other recombinant APC). It was also found that the APC treatment increased the bleeding risk in patients with sepsis, highlighting the importance of measuring the levels of PROC and/or APC in patients before and during the treatment.

Thus, rhAPC (Drotrecogin alpha, DrotAA) particularly Xigris^{®} (Eli Lilly Inc.) was withdrawal from the market by the manufacturer and efforts were made to improve the therapeutic potential and safety of APC treatment in several diseases. To decrease bleeding risks, the native APC structure was mutated: 3 lysines were replaced by alanine (K191A, K192A, K193A), removing >90% of the anticoagulant activity while preserving multiple cell signaling activities. This APC form was named 3K3A-APC. ZZ Biotech's 3K3A-APC (http://zzbiotech.com/the-company/3k3a-activated-protein-c) is thus a genetically engineered variant of the naturally occurring APC, which plays a role in the regulation of blood clotting and inflammation. APC has cell-protecting, anti-inflammatory and anti-coagulant properties; 3K3A-APC has reduced anti-coagulant ability, which minimizes the risk of bleeding induced by unmodified APC. In animal models of stroke, amyotrophic lateral sclerosis (ALS), neurotrauma, and sepsis, 3K3A-APC therapy has shown an advantage over recombinant APC in enhanced efficacy and reduced risk for bleeding. The protective effect of 3K3A-APC on the lining of blood vessels in the brain further helps prevent bleeding sometimes caused by tissue plasminogen activator, or tPA, the only drug currently indicated for the treatment of acute ischemic stroke and has been recently proposed for seriously ill patients with coronavirus disease 2019.

Previous studies on the therapeutic applications of recombinant natural or mutated human APC reinforce the idea of the need of detection and monitorization methods for measuring endogenous and exogenous human PROC or/and APC, and also for diagnostic and therapeutic settings. The present invention describes methods to measure circulating PROC and APC in blood obtained from human subjects, including in plasma of patients admitted at the Intensive Care Unit of tertiary hospitals (including severe affected patients with sepsis and septic shock, and cerebrovascular accidents like stroke). Methods described herein are based on multiple reaction monitoring targeted mass spectrometry (MRM-MS) and the use of specific (spiked-in labelled) proteotypic peptides, obtaining a highly sensitive and specific method.

### DESCRIPTION OF THE FIGURES

**Figure 1****.** Linearity for the selected peptides of APC using different concentrations (fmol) of heavy spike-in peptides in MS experiments.
**Figure** 2. Spearman correlation between APC (ng/mL) and different parameters related with the physiopathology of sepsis. APC correlation with a) histone H2B (Spearman's Rho 0.469, p = 0.021), b) Procalcitonin (PCT) (Spearman's Rho 0.468, p = 0.021), c) Quick index (Spearman's Rho 0.514, p = 0.010), d) Functional protein C (Spearman's Rho 0.900, p = 0.037).
**Figure 3****.** Spearman correlation between APC (ng/mL) and different parameters related with the physiopathology of septic shock. APC correlation with: a) platelets (Spearman's Rho 0.374, p = 0.0001), b) Quick index (Spearman's Rho 0.302 p = 0.003), c) activated partial thromboplastin time (Spearman's Rho -0.267, p = 0.026), d) D-dimer (Spearman's Rho -0.437, p = 0.002) and e) functional protein C (Spearman's Rho 0.900 rho, p <0.0001).
**Figure 4****.** Study of the plasmatic APC levels in samples of patients with diagnosed CID or without CID (p=0.0014).

### BRIEF DESCRIPTION OF THE INVENTION

In a first aspect, the invention relates to a mass spectrometry-based method for measuring the levels of Protein C (PROC), preferably Activated Protein C (APC), in a subject in need thereof, the method comprising a step of performing an enzymatic digestion to one or more biological samples isolated from the subject, and a step of measuring by using a mass spectrometer the levels of the peptide of SEQ ID NO 1 (LGEYDLR), wherein the levels of SEQ ID NO 1 (LGEYDLR) correspond to the levels of PROC, preferably APC, present in said one or more biological samples.

The mass spectrometry-based method according to the first aspect, wherein the enzyme used for the enzymatic digestion is trypsin and/or ArgC, or any combination of proteases which give rise after digestion to the LGEYDLR peptide of SEQ ID NO 1.

The mass spectrometry-based method according to the first aspect or any of the previous aspects, wherein the measurement of the peptide of SEQ ID NO 1 (LGEYDLR) is carried out by spiking-in a labelled proteotypic peptide of SEQ ID NO 1 (LGEYDLR).

The mass spectrometry-based method according to the first aspect or any of the previous aspects, wherein the spike-in labelled proteotypic peptide of SEQ ID NO 1 (LGEYDLR) is synthesized with at least one or more heavy amino acids and wherein said peptide is preferably labelled in the C-terminal amino acid residue with a tag.

The mass spectrometry-based method according to the first aspect or any of the previous aspects, wherein the method further comprises a step of reducing the one or more biological samples with a reducing agent, and a step of alkylating the one or more biological sample with an alkylating agent, wherein both the reduction and the alkylation steps are performed before the step of the enzymatic digestion.

The mass spectrometry-based method according to the first aspect or any of the previous aspects, wherein the one or more biological samples are selected from the group consisting of blood, serum, plasma, saliva, urine or cerebrospinal fluid.

A second aspect of the present invention relates to a mass spectrometry-based method for screening or classifying subjects at risk of having sepsis or septic shock, the method comprising the steps of:
i) measuring the levels of Protein C (PROC), preferably Activated protein C (APC), by carrying out the method of the first aspect or any of the previous aspects, and
ii) comparing the levels obtained in step i) with a reference value,
wherein a statistically significant variation of the measurement of step i) with the reference value of ii) is indicative that the subject is at risk of having sepsis or septic shock.

The mass spectrometry-based method according to the second aspect, wherein the reference value of step ii) is obtained from a healthy subject or a population of healthy subjects and wherein a statistically significant reduction in the levels of Protein C, preferably Activated Protein C, is indicative that the subject is at risk of having sepsis or septic shock.

The mass spectrometry-based method according to the second aspect or any of the previous aspects, wherein the method further comprises a step of clinical confirmation that the patient has sepsis or septic shock.

Further, the second or any of the previous aspects also relates to an *in vitro* use of the levels of peptide of SEQ ID NO 1 (LGEYDLR) in one or more biological samples isolated from subjects in need thereof as a biomarker for screening for subjects at risk of having sepsis or sepsis shock.

A third aspect of the present invention relates to a mass spectrometry-based method for screening or classifying subjects at risk of having disseminated intravascular coagulation (DIC), wherein the subjects have been diagnosed with sepsis or septic shock, the method comprising the steps of:
i) measuring the levels of Protein C (PROC), preferably of Activated protein C (APC), by carrying out the methods of the first aspect or any of the previous aspects, and
ii) comparing the levels obtained in step i) with a reference value,
wherein a statistically significant variation of the measurement of step i) with the reference value of ii) is indicative that the subject is at risk of having DIC.

The mass spectrometry-based method according to the third aspect, wherein the reference value of step ii) is obtained from a subject or a population of subjects having sepsis or septic shock but not having disseminated intravascular coagulation (DIC) and wherein a statistically significant reduction in the levels of Protein C (PROC), preferably Activated Protein C (APC), is indicative that the subject is at risk of having DIC.

The mass spectrometry-based method according the third aspect or any of the previous aspects, wherein the method further comprises a step of clinical confirmation that the patient has disseminated intravascular coagulation.

A fourth aspect of the present invention relates to a mass spectrometry-based method for prognosticating the response to treatment with recombinant Protein C (PROC), preferably recombinant Activated Protein C (APC), in a subject in need thereof, the method comprising the steps of:
i) screening if the subject is at risk of having disseminated intravascular coagulation (DIC) by carrying out each of the steps of the method of the third aspect or any of the previous aspects,
ii) classifying the subject as responder to said treatment if the subject is identified at risk or diagnosed with DIC and as non-responder to said treatment if the subject is not identified at risk nor diagnosed with DIC.

The method of the fourth aspect, wherein the prognosis is performed prior treating the subject with recombinant Protein C (rPROC), preferably recombinant Activated Protein C.

The fourth aspect also relates to an *in vitro* use of the levels of peptide of SEQ ID NO 1 (LGEYDLR) in one or more biological samples isolated from subjects having sepsis or septic shock as a biomarker for screening for subjects at risk of having disseminated intravascular coagulation (DIC).

Further, the fourth aspect also relates to an *in vitro* use of the levels of peptide of SEQ ID NO 1 (LGEYDLR) in one or more biological samples isolated from subjects in need thereof as a biomarker for prognosticating the response to treatment with recombinant PROC, preferably recombinant APC, in a subject having sepsis or septic shock and disseminated intravascular coagulation (DIC).

A fifth aspect of the present invention relates to a composition comprising recombinant Protein C (rPROC), preferably recombinant Activated Protein C (rAPC), for use in the treatment of sepsis, septic shock or stroke wherein the use comprises the step of administering said composition to the subjects that are classified as responders according to the fourth aspect or to any of the previous aspects.

### DESCRIPTION OF THE INVENTION

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

As used herein, the term "about" means the indicated value ± 1 % of its value, or the term "about' means the indicated value ± 2% of its value, or the term "about" means the indicated value ± 5% of its value, the term "about" means the indicated value ± 10% of its value, or the term "about" means the indicated value ± 20% of its value, or the term "about" means the indicated value ± 30% of its value; preferably the term "about" means exactly the indicated value (± 0%).

When used herein "consisting of" excludes any element, step, or ingredient not specified in the claim element. When used herein, "consisting essentially of does not exclude materials or steps that do not materially affect the basic and novel characteristics of the claim.

In the context of the present invention, the terms "detection", "quantification" and "measurement" are considered synonymous and refer to calculating the levels of PROC, preferably APC, in the one or more biological samples. The term "levels" as used herein is synonymous of concentration or quantity. Thus, for instance, the measurement of the levels of PROC, preferably APC, in the context of the present invention is meant that the concentration or quantity of said proteins is calculated.

As used herein, the term "sepsis" is defined as a suspected or proven infection plus a systemic inflammatory response syndrome (for example, fever, tachycardia, tachypnea, or leukocytosis). Importantly, in many cases of sepsis, it is not possible to establish the cause of an infection.

As used herein, the term "septic shock" is defined as a serious condition that occurs when a systemic infection leads to dangerously low blood pressure. Septic shock is usually related to a significant drop in blood pressure that can lead to respiratory or heart failure, stroke, failure of other organs, and death.

As used herein, the term "disseminated intravascular coagulation (DIC)" is defined as an acquired clinic biological syndrome characterized by widespread activation of coagulation leading to fibrin deposition in the vasculature, organ dysfunction, consumption of clotting factors and platelets, and life-threatening haemorrhage.

As used herein, the term "Protein C deficiency" is defined as a genetic disorder characterized by a deficiency of protein C, so subjects affected by this rare disease are at risk for developing clots, particularly a type of blood clot called deep vein thrombosis.

As used herein, the term "Protein C" (PROC) is defined as vitamin K-dependent glycoprotein that is the zymogen (inactive precursor of an enzyme) of activated protein C (APC). As used herein, activated protein C (APC) is defined as a 56-kDa blood vitamin K-serine protease resulted from the cleavage of an Arg169-Leu170 peptide bond from the Protein C, releasing a dodecapeptide (residues 158-169) from the heavy chain.

As used herein, the term "endogenous protein" is defined as a protein that is originated in the subject. Endogenous protein contracts with "exogenous proteins" in that exogenous proteins have been originated outside the subject, for instance, they come from another subject. Thus, endogenous PROC or APC is referred to the PROC or APC synthetized by the subject, while exogenous PROC or APC is referred to the PROC or APC that are not synthetized by the subject but have been administrated to it, which can thus be recombinant (i.e., generated by laboratory methods) PROC or APC.

### DESCRIPTION

Drotrecogin alfa is a recombinant form of human activated protein C. Xigris^{®} (by Eli Lilly) is a medicament which contains the active substance drotrecogin alfa (activated), and that was authorised in the European Union under exceptional circumstances in 2002 for the treatment of severe sepsis in adult patients with multiple organ failure, in addition to best standard care.

Later on, the results of the PROWESS-SHOCK study using Xigris^{®} failed to meet the primary endpoint of a statistically significant reduction in 28-day all-cause mortality in patients treated with Xigris compared with placebo. The study also failed its secondary endpoint of a reduction of mortality in the population of patients with severe protein C deficiency. Eli Lilly decided to withdraw the product from the market worldwide.

Newly developed optimized medicaments based on rhAPC have been developed, including 3K3A-APC, disclosed in US2010028910, with apparently substantial reduction in anticoagulant activity. Further, rhAPC therapy has also been proposed for treatment of stroke. However, due to the strong evidence that showed that APC treatment is associated with increased risk of serious bleeding, there is an important uncertainty regarding the outcome of APC-related treatments.

Due to the important secondary effects associated to recombinant PROC or APC treatment, there is a need for the replacement of currently used methods to measure the levels of endogenous PROC and APC in patients by other methods or techniques with increased sensitivity and specificity, in order to improve the classification of subjects at risk of having sepsis or sepsis shock or DIC, and to prognosticate the response to recombinant PROC (rPROC) or recombinant APC (rAPC) treatment.

The present invention aims to solve the above problems by providing efficient and accurate methods for measuring PROC and APC based on the detection using mass-spectrometry of two specific peptides LGEYDLR (SEQ ID NO. 1) and TFVLNFIK (SEQ ID NO 2) that have been found by the authors of the present invention to correlate with the levels of endogenous PROC and APC. Further, the present invention also provides compositions comprising PROC or APC for use in treatment of said diseases as well as for patient selection. Lastly, *in vitro* uses of peptides LGEYDLR and TFVLNFIK as biomarkers are also provided.

### Methods for detecting PROC, preferably APC.

**A first aspect** of the present invention relates to a method for measuring the levels of Protein C (PROC), preferably Activated Protein C (APC), in a subject in need thereof, the method comprising a step of performing an enzymatic digestion of one or more biological samples isolated from the subject, and a step of measuring the levels of the peptide of SEQ ID NO 1 (LGEYDLR) and/or SEQ ID NO 2 (TFVLNFIK), wherein the levels of SEQ ID NO 1 and/or SEQ ID NO 2 correspond to the levels of PROC, preferably APC, present in said one or more biological samples.

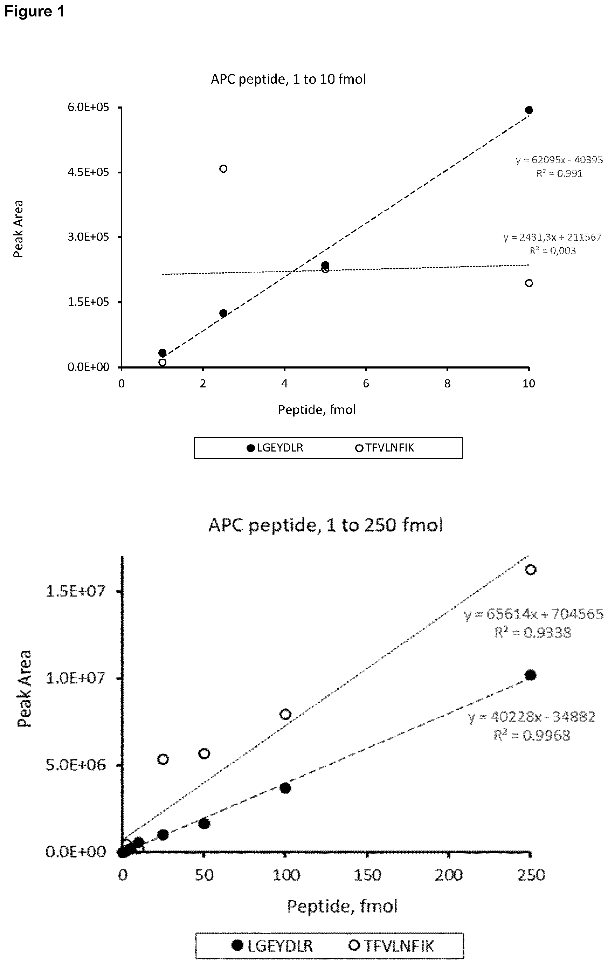

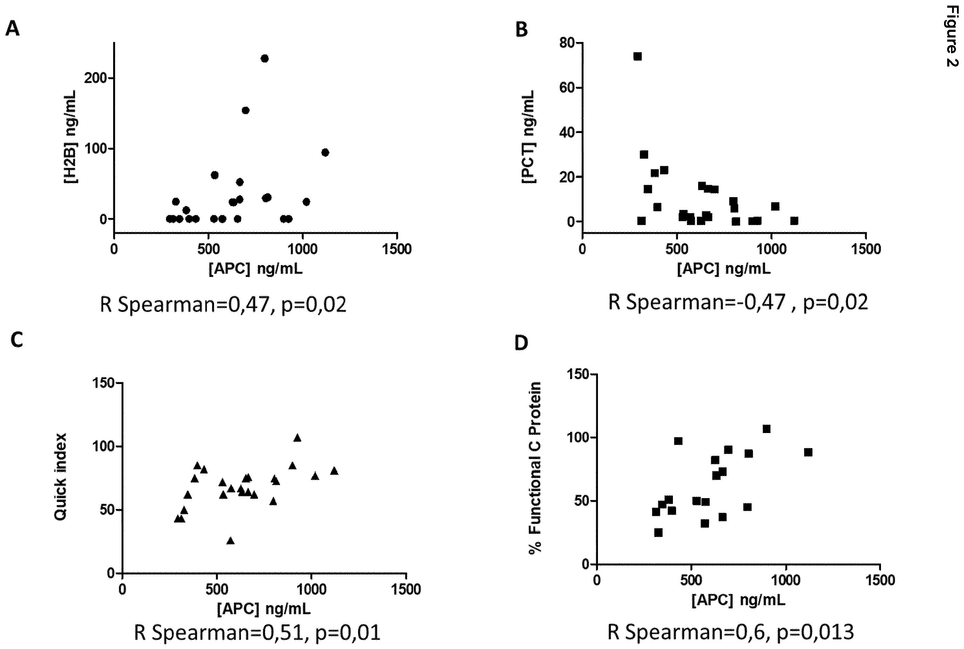

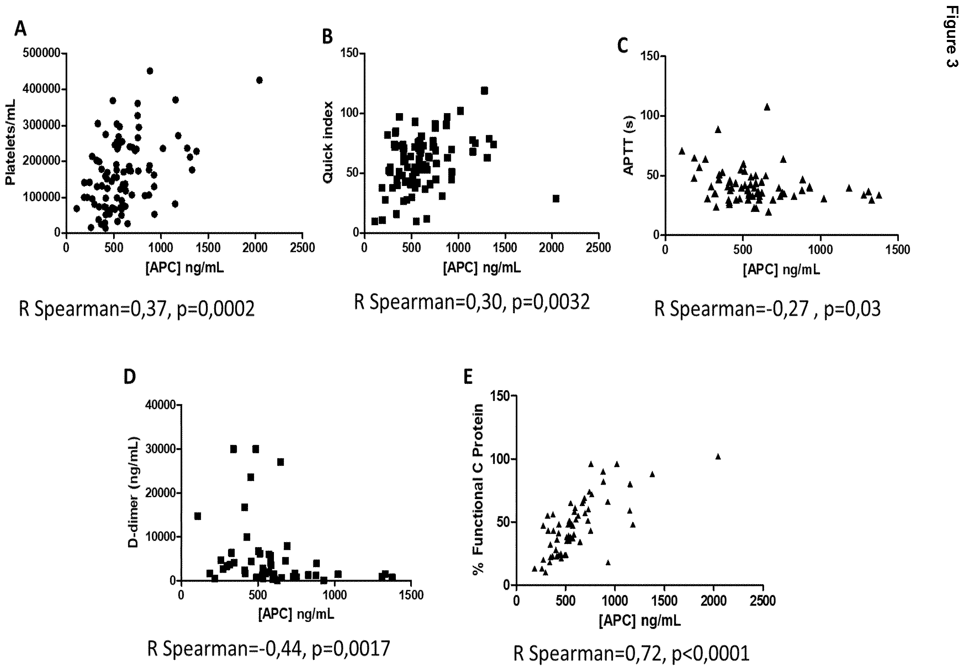

In order to provide such methodology and select the peptides that have a significant correlation with the levels of PROC, preferably of APC, two peptides (SEQ ID NO 1: LGEYDLR and SEQ ID NO 2: TFVLNFIK) were selected and tested for their linearity with said proteins. It was a finding of the present invention that the levels of the peptide of SEQ ID NO 1 and 2 can be used to calculate the levels of PROC, preferably APC. This is possible because there is a correlation between peptide SEQ ID NO 1 and 2 and PROC and APC, as shown in Fig. 1. Further, it was also found that, among both peptides, the peptide of SEQ ID NO 1 showed better linearity with the levels of PROC and APC than SEQ ID NO 2, making this peptide the preferred peptide to measure the levels of PROC, preferably APC, in one or more biological samples. Lastly, it was also found that Functional Protein C also correlates with concentration of APC determined by mass spectrometry in groups having sepsis or sepsis shock (see Figs. 2 and 3). Importantly, PROC/APC also correlate, in septic patients, with the levels of histone H2B (a cell-injury mediator when is released from neutrophiles), PCT (procalcitonin which is elevated in the cases of active infection), Quick index (which is a parameter related with blood coagulation). In the group of septic shock patients, PROC/APC levels correlated with platelets (involved in coagulation process), activated thromboplastin time (which is used to evaluate a person's ability to appropriately form blood clots) and D-dimer (which levels helps to rule out the presence of a serious blood clot).

Thus, in an embodiment, the levels of the peptide of SEQ ID NO 1 and/or SEQ ID NO 2, preferably SEQ ID NO 1 (LGEYDLR), correlate linearly with the levels of PROC, preferably APC, present in said one or more biological samples. In another embodiment, said linear correlation lies between -1.0 and 1.0 and it relates the variables through an equation of the form Y = bX, wherein X corresponds to the levels of the peptides of SEQ ID 1 and/or SEQ ID NO 2, Y corresponds to the levels of PROC, preferably, APC, and b is 1.

The enzymatic digestion step of the first aspect or any of its embodiments is very important for the method. This step is performed before the measuring step since the one or more biological samples need to be transformed or digested into separated peptides prior measurement. Examples of enzymes that can be used for the enzymatic digestion are trypsin, chymotrypsin, pepsin, papain pepsin, papain, Staphylococcus aureus (V8) protease, Submaxillaris protease, bromelain, thermolysin, aspartate endopeptidase, or ArgC, or any combination thereof. Preferably, the enzymes used for the enzymatic digestion are enzymes that digest PROC and APC proteins giving rise to a number of peptides where the peptides of SEQ ID NO 1 and/or 2, are included. In a preferred embodiment, trypsin, ArgC and/or any combination of proteases which give rise after digestion to the peptide of SEQ ID NO 1 and/or SEQ ID NO 2 are used for the enzymatic digestion step. Optimal digestion conditions such as time and temperature are known in the art.

Optionally, microwave-assisted digestion could be used during the enzymatic digestion to accelerate the formation of peptides (P Muralidhar Reddy, et al. Digestion Completeness of Microwave-Assisted and Conventional Trypsin-Catalyzed Reactions. Journal of the American Society for Mass Spectrometry. Volume 21, Issue 3, March 2010, Pages 421-424). In a preferred embodiment, the enzymatic digestion is a microwave assisted digestion with trypsin and/or ArgC and/or any combination of proteases which give rise after digestion the peptide of SEQ ID NO 1 and/or SEQ ID NO 2.

Further, even before the enzymatic digestion, the one or more biological samples can optionally be treated with a reducing agent and/or with an alkylating agent. By "reducing agent" is meant any element or compound that loses (or donates) an electron to an electron recipient in a redox chemical reaction, resulting in an oxidation of the reducing agent and a reduction of the recipient compound. Examples of reducing agents are dithiothreitol (DTT), Tris (2-carboxyethyl phosphine (TCEP), ascorbic acid, cysteine hydrochloride, 2-mercaptoethanol, sodium sulfite, or sodium thioglycolate. By "alkylating agent" is meant any element or compound that transfers an alkyl group to another compound. An example of an alkylating agent used for peptide mapping purposes is iodoacetamide.

Thus, in a further preferred embodiment of the first aspect, the method can further comprise a step of reducing the one or more biological samples with a reducing agent, and/or a step or alkylating the one or more biological sample with an alkylating agent, wherein both the reduction and/or the alkylating steps are performed before the step of the enzymatic digestion and thus before the measurement step.

In an embodiment, the one or more biological samples used to measure the levels of PROC, preferably APC, are selected from the group consisting of blood, serum, plasma, saliva, urine or cerebrospinal fluid. Preferably, the one or more biological samples are selected from the group consisting of blood, serum, or plasma.

In an embodiment, the subject according to the first aspect or any of its preferred embodiments is a mammal, preferably a human. In another embodiment, the human subject is selected from patients that are at the intensive care unit (ICU).

It should be noted that the method described in the first aspect or any of its embodiments is able to measure both endogenous or exogenous Protein C, and activated protein C (APC), since all of these protein variants comprise the peptides of SEQ ID NO 1 and 2. Further, the method of the first aspect of the present invention or any of its embodiments can also measure any fragment or variant of said proteins as long as the fragment or variant comprise the measured peptides of SEQ ID 1 and/or SEQ ID NO 2. Therefore, in an embodiment of the first aspect, the measured PROC or APC is from exogenous and/or endogenous origin. In another embodiment, the measured PROC, preferably APC, is the exogenous 3K3A-APC or the activated drotrecogin alfa comprised in Xigris^{®} medicament.

Although the peptide of SEQ ID NO 2 (TFVLNFIK) did not show as good linearity as the peptide of SEQ ID NO 1 (LGEYDLR), in an embodiment of the present invention, both peptides can be combined in order to obtain a more robust method that includes both measurements to provide a better calculation of the levels of PROC, preferably APC. Alternatively, they can be used separately. In a preferred embodiment of the first aspect or any of its embodiments, the measurement step is performed with only the peptide of SEQ ID NO 1.

Regarding the techniques used in measurement step present in the first aspect or any of its embodiments, any technique can be used as long as it specifically detects the peptides of SEQ ID NO 1 and/or SEQ ID NO 2 since, as stated above and shown in Fig. 1, these peptides correlate with the levels of PROC and APC. Thus, for instance, the technique used for the measurement step may be radioimmunoassay (RIA), enzyme linked immunosorbent assay (ELISA), chemiluminescent or colorimetric ELISA, immunofluorescence assay (IFA), dot blot, western blot, flow cytometry, Luminex assay and ProQuantum immunoassay, or, preferably, mass-spectrometry.

In a preferred embodiment, the first aspect relates to a mass spectrometry-based method for measuring the levels of Protein C (PROC), preferably Activated Protein C (APC), in a subject in need thereof, the method comprising a step of performing an enzymatic digestion of one or more biological samples isolated from the subject, and a step of measuring by using a mass spectrometer the levels of the peptide of SEQ ID NO 1 and/or SEQ ID NO 2, wherein the levels of SEQ ID NO 1 and/or SEQ ID NO 2 correspond to the levels of PROC, preferably APC, present in said one or more biological samples.

In a further preferred embodiment, a method based on Liquid chromatography with tandem mass spectrometry (LC-MS/MS) and multiple reaction monitoring approach (MRM) (MRM-MS) is used. MRM-MS offers a potential methodology for both biomarker discovery and biomarker validation. In addition, this method provides the sensitivity, accuracy and high throughput needed for clinical validation of biomarkers, thus eliminating the necessity of performing different discovery and validation methods. Plasma is a source of biomarkers for diagnostic assays, but it is also a challenging biological matrix for proteomic studies because of the wide dynamic range of proteins present in this fluid (10¹⁰). Monitoring of a single transition per peptide has been proposed as being sufficiently specific for peptide quantitation in complex mixtures such as plasma. On the other hand, classical biomarkers do not add more information than generic scores in predicting outcome in septic patients. Despite having C Reactive Protein (CRP) and procalcitonin (PCT) to complement the diagnosis of sepsis, and the usefulness of the PCT as a prognostic biomarker, the future assessment of sepsis requires finding of new biomarkers that can be sensitive, specific, and economically affordable.

Preferably, the mass spectrometer of the first aspect is a triple quadrupole or hybrid triple quadrupole-linear ion trap mass spectrometer, preferably equipped with a chromatographic system, more preferably equipped with a nano-LC or micro-LC chromatographic system. Preferably, the mass spectrometry-based method for measuring PROC, preferably APC, in a subject according to the first aspect of the invention, the level or concentration of PROC, preferably APC, is equivalent to the amount of peptide SEQ ID NO 1 and/or SEQ ID NO 2. Preferably, to measure the level or concentration of PROC or APC in the biological sample, the averaged ratio, understood as the ratio signal peptide of interest/signal peptide spike-In, of SEQ ID NO 1 and/or SEQ ID NO 2 is obtained. More preferably, the selected peptide sequence of SEQ ID NO 1 and/or SEQ ID NO 2 can be preferably prepared as SpikeTidesTM_TQ (heavy-isotope labeled such as Arg: ¹³C6, ¹⁵N4; Lys: Arg: ¹³C6, ¹⁵N2) for absolute quantification of PROC, preferably APC, in the method according to the first aspect of the invention or to any of its preferred embodiments.

In a preferred embodiment, the measurement of PROC, preferably APC, by measuring the peptide of SEQ ID NO 1 and/or SEQ ID NO 2 is carried out by spiking-in a labelled proteotypic peptide of SEQ ID NO 1 and/or SEQ ID NO 2. In a further preferred embodiment, the spike-in labelled proteotypic peptide of SEQ ID NO 1 and/or SEQ ID NO 2 is synthesized with at least one or more heavy amino acids and wherein said peptide is preferably labelled in the C-terminal amino acid residue with a tag.

In the context of the present invention, the term "tag" is understood as stable isotope labelled amino acids which share the same physiochemical properties and the same chemical activity with their non-labelled amino acids counterparts. Tags can also be a small chemical modification which is cleaved during trypsin or other proteases treatment previous MS experiments. Tags linked to proteotypic peptides are used for peptide quantification in targeted MS experiments.

In the context of the present invention, the term "heavy amino acids" refers to isotopic atoms which are incorporated into the tag containing heavy atoms for example ¹³C, ¹⁵N, ¹⁷O and/or ³⁴S, which can be distinguished by MS.

In the context of the present invention, the term "synthesized with at least one or more heavy amino acids" is understood as the "Tag" containing at least one or more heavy atoms ¹³C, ¹⁵N, ¹⁷O and/or ³⁴S in the Spike-In amino acids sequence corresponding to SEQ ID NO 1 and/or SEQ ID NO 2.

### Methods for screening or classifying patients with sepsis or sepsis shock

The method as defined in the first aspect or in any of its embodiments also allows the screening or classification of patients at risk of having sepsis or sepsis shock according to the levels of PROC, preferably APC, measured in said patients. As shown in the examples, specifically in Table 2, 157 plasma samples were analyzed and statistically significant differences were found between patients with sepsis or sepsis shock versus patients not suffering from these diseases. Particularly, these differences were statistically significant when the levels of PROC, preferably APC, were below 800 ng/ml, indicating that measuring the levels of PROC, preferably APC, using the method as defined in the first aspect or in any of its embodiments could be useful for screening and classifying patients at risk of suffering said diseases.

In view of this, a **second aspect** of the present invention refers to a mass spectrometry-based method, preferably a method based on multiple reaction monitoring targeted mass spectrometry (MRM-MS) and multiple reaction monitoring approach, for screening or classifying subjects at risk of having sepsis or septic shock, the method comprising the steps of:
i) measuring the levels of Protein C (PROC), preferably Activated protein C (APC), by carrying out the method as defined in the first aspect or in any of its embodiments, and
ii) comparing the levels obtained in step i) with a reference value,
wherein a statistically significant variation of the measurement of step i) with the reference value of ii) is indicative that the subject is at risk of having sepsis or septic shock.

As indicated in the first aspect, the measurement step can be performed with any analytical technique that allows quantification of the peptides of SEQ ID NO 1 and/or SEQ ID NO 2, wherein mass spectrometry is preferred. Thus, although the second aspect and any of the following embodiments specifically refer to mass spectrometry, it is to be understood that this technique can be substituted by any other technique, such as radioimmunoassay (RIA), chemiluminescent or colorimetric enzyme linked immunosorbent assay (ELISA), immunofluorescence assay (IFA), dot blot, western blot, flow cytometry or, preferably, mass-spectrometry.

In a preferred embodiment, the reference value of step ii) of the second aspect is obtained from a healthy subject or a population of healthy subjects (the reference value is in this case an average value). Alternatively, the reference value of the second aspect is obtained from a subject or a population of subjects that are at the intensive care unit (UCI) but do not suffer from sepsis or septic shock. To obtain said reference value, the method described in the first aspect of the invention or in any of its embodiments can be used. Alternatively, other methods for measuring the levels of PROC, preferably APC, in a healthy subject or a population of healthy subjects known in the art can be used.

Also preferably, the statistically significant variation of the levels measured in step i) (the levels of PROC, preferably of APC) is a statistically significant reduction in comparison with the reference value. This significant reduction is indicative that the subject is at risk of having sepsis or septic shock. The significant reduction is defined as a decrease in the levels of peptide of SEQ ID NO 1 and/or SEQ ID NO 2, which in turns means a significant decrease in PROC or APC, in the biological sample when said levels have been compared to reference levels by means of a statistic test. The statistic test can be parametric or, preferably, non-parametric, and the significant threshold, defined as the threshold to discriminate significant from non-significant results, can be set up as lower or equal to 0.05, lower or equal to 0.005, or lower or equal to 0.001.

It is estimated that average amount of circulating PROC or APC in human body is between 3.000-5.000 ng/ml (see, for instance, https://diapharma.com/protein-c/, https://emedicine.medscape.com/article/2085992-overview). In a particular embodiment, the reference value for PROC, preferably APC, is about 4000 ng/mL, wherein a reduction of at least about 10%, of at least about 20%, of at least about 30%, preferably of at least about 40%, of at least about 45%, of at least 50%, of at least about 60%, of at least about 70%, of at least about 80%, of at least about 90%, of at least 95%, of at least 100%, is indicative of being at risk of sepsis or septic shock. As previously defined in the definitions section, the term "about" can refer the indicated value ± 10%.

As shown in Table 2, the significant reduction obtained by Mann Whitney U test (non-parametric) that indicated a risk of having sepsis or septic shock was levels of APC of 613,70 ng/ml in comparison with the control levels 1431.57 ng/ml (reference value). This reduction corresponds to about a 40% (0.4 fold) reduction to the reference level.

Thus, in a preferred embodiment, the reference value for PROC, preferably APC, is about 1431.57 ng/mL, wherein a reduction of at least about 10%, of at least about 20%, of at least about 30%, preferably of at least about 40%, of at least about 45%, of at least 50%, of at least about 60%, of at least about 70%, of at least about 80%, of at least about 90%, of at least 95%, of at least about 100%, is indicative of being at risk of sepsis or septic shock. As previously defined in the definitions section, the term "about" can refer the indicated value ± 10%.

Further, as also shown in the ROC curve (Table 4, Example 5) obtained from comparing subjects with sepsis or sepsis shock in subjects versus controls (reference population + ICU controls), optimal cut-off value for said reduction was calculated as about 800 ng/ml, with a sensitivity of 79.5% and a specificity of 97.1% (p<0.0001). Thus, in another embodiment, the significant reduction is referred to a reduction of at least below levels of 800 ng/ml of PROC, preferably APC, selected as cut-off value according to the ROC curves exemplified herein.

It is noted that, once the method of the second aspect of the present invention or any of its preferred embodiments has been carried out and the subjects at risk of having sepsis or septic shock have been identified, it is preferable that a clinical confirmation method, such as CRP or PCT, is performed in order to confirm whether the identified subjects have indeed sepsis or septic shock. Thus, in a preferred embodiment, the method of the second aspect or any of its embodiments further comprises a step of clinical confirmation that the patient has sepsis or septic shock.

In a further embodiment, the present invention also relates to the *in vitro* use of the levels of the peptide of SEQ ID NO 1 and/or SEQ ID NO 2 in one or more biological samples isolated from subjects in need thereof as a biomarker for screening for subjects at risk of having sepsis or sepsis shock.

In another particular embodiment, the method according to the second aspect or any of its embodiments is also useful to classify patients at risk of having sepsis or septic shock in the two sub-phenotypes (endotypes), which are sepsis response signature 1 (SRS1) and 2 (SRS2). This endotype classification is important for the treatment of choice, particularly in the corticosteroids treatment as described by Antcliffe and Gordon 2019. [David B. Antcliffe, Anthony C. Gordon. 2019 Why Understanding Sepsis Endotypes Is Important for Steroid Trials in Septic Shock. Society of Critical Care Medicine and Wolters Kluwer Health, Inc. Volume 47. Number 12. DOI: 10.1097/CCM.0000000000003833].

### Methods for screening or classifying patients with DIC

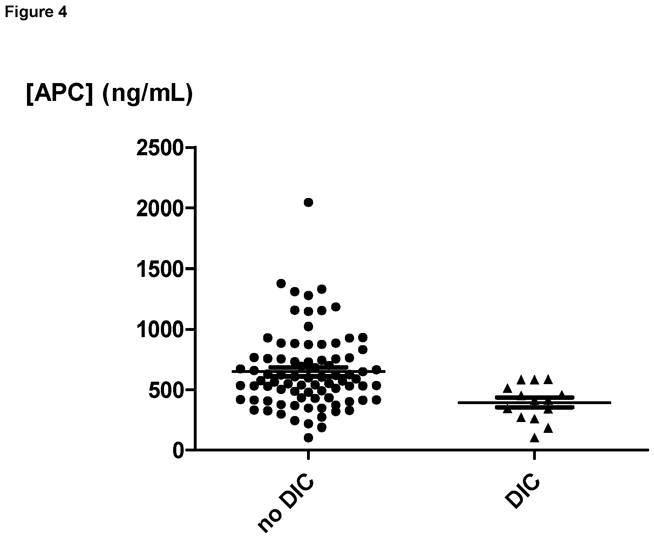

As mentioned above, previous studies have shown that treatment with recombinant APC did not have any effect in the majority of patients with sepsis. However, interestingly, only patients suffering from disseminated intravascular coagulation (DIC) did respond to recombinant APC treatment. Thus, future studies regarding the safety and efficacy of recombinant APC-related treatments will need to identify patients with sepsis or sepsis shock and at risk of having DIC, since they may in turn be the real responders to said treatment. In this regard, it has been found in the present invention that the mean levels of APC in patients with diagnosed DIC are lower than those not suffering from DIC, as shown in Fig. 4. In particular, it was found that levels of PROC, preferably APC, below 468 ng/mL indicate that the patient suffering from sepsis or sepsis shock is at risk of having DIC.

In view of this finding, a **third aspect** of the invention refers to a mass spectrometry-based method for screening or classifying subjects at risk of having disseminated intravascular coagulation (DIC), the method comprising the steps of:
i) measuring the levels of Protein C (PROC), preferably of Activated protein C (APC), by carrying out the methods as defined in the first aspect or in any of its embodiments, and
ii) comparing the levels obtained in step i) with a reference value,
wherein a statistically significant variation of the measurement of step i) with the reference value of ii) is indicative that the subject is at risk of having DIC.

As indicated in the first and second aspects, the measurement step can be performed with any analytical technique that allows quantification of the peptides of SEQ ID NO 1 and/or 2, wherein mass spectrometry is preferred. Thus, although the third aspect and any of the following embodiments specifically refer to mass spectrometry, it is to be understood that this technique can be substituted by any other technique, radioimmunoassay (RIA), chemiluminescent or colorimetric enzyme linked immunosorbent assay (ELISA), immunofluorescence assay (IFA), dot blot, western blot, flow cytometry, Luminex assay, ProQuantum immunoassay or, preferably, mass-spectrometry.

In a preferred embodiment of the third aspect, the reference value of step ii) is obtained from a subject or a population of subjects having sepsis or septic shock but not having disseminated intravascular coagulation (DIC). This reference value can be obtained from said subjects by performing the method according to the first or the second aspect or any of their embodiments.

Also preferably, the statistically significant variation of the levels measured in step i) (the levels of PROC, preferably of APC) is a statistically significant reduction in comparison with the reference value. This significant reduction is indicative that the subject is at risk of having DIC. The statistically significant variation can be calculated by means of statistical tests as defined previously for the second aspect or any of its embodiments, but considering that, in this case, the reference value comes from a different subject or population of subjects (that have sepsis or septic shock) than in the second aspect (that are healthy or that are at ICU but not suffering from sepsis).

As shown in Fig. 4, the APC levels in samples of patients diagnosed with DIC (average 468 ng/ml) are statistically reduced (p=0.0014) in comparison with the levels of subjects without DIC (average 600 ng/ml). This reduction corresponds to about 20% (0.2 fold) reduction to the reference level.

Thus, in an embodiment, the reference value for PROC, preferably APC, of patients having sepsis or septic shock but not suffering from DIC is about 600 ng/mL, wherein a reduction of at least about 10%, preferably of at least about 20%, of at least 25%, of at least about 30%, of at least about 40%, of at least about 45%, of at least 50%, of at least about 60%, of at least about 70%, of at least about 80%, of at least about 90%, of at least 95%, of at least about 100% is indicative of being at risk of having DIC. As previously defined in the definitions section, the term "about" can refer the indicated value ± 10%.

Further, as shown in Example 6, the ROC curves (Table 5) for the identification of DIC in subjects with sepsis, provided an optimal cut-off value for said reduction of about 468 ng/ml, with a sensitivity of 71.4% and a specificity of 71.4% (p=0.00013). Thus, in another embodiment, the significant reduction that is indicative that the patient is at risk of having DIC is referred to a reduction of at least below the levels of 468 ng/ml of PROC, preferably APC, selected as cut-off value according to the ROC curves exemplified herein.

In a preferred embodiment, the subject has been previously diagnosed with sepsis or septic shock prior performing the method according to this aspect or any of its embodiments.

Similarly as defined in the second aspect, once the method of the third aspect of the present invention or any of its preferred embodiments has been carried out and the subjects is classified at risk of having DIC, it is preferable that a clinical confirmation method is performed in order to confirm whether the identified subjects have DIC. Thus, in a preferred embodiment, the method of the third aspect further comprises a step of clinical confirmation that the patient has DIC.

Methods for prognosticating the response to treatment with exogenous PROC, preferably recombinant PROC (rPROC), and exogenous APC, preferably recombinant APC (rAPC).

As explained above, in order to assess the individual patient response to recombinant PROC or APC treatment, it is important to know whether the subject being treated or to be treated is at risk of having DIC. In line with this, a **fourth aspect** of the present invention relates to a mass spectrometry-based method for prognosticating the response to treatment with recombinant Protein C (rPROC), preferably recombinant Activated Protein C (rAPC), in a subject in need thereof, the method comprising the steps of:
i) screening if the subject is at risk of having disseminated intravascular coagulation (DIC) by carrying out each of the steps of the method as defined in the third aspect or in any of its embodiments, and
ii) classifying the subject as responder to said treatment if the subject is identified at risk of having DIC or diagnosed with DIC and as non-responder to said treatment if the subject is not identified at risk nor diagnosed with DIC.

As indicated in the first, second and third aspects, or in any of its embodiments, the measurement step can be performed with any analytical technique that allows quantification of the peptides of SEQ ID NO 1 and/or SEQ ID NO 2, wherein mass spectrometry is preferred. Thus, although the fourth aspect and any of the following embodiments specifically refer to mass spectrometry, it is to be understood that this technique can be substituted by any other technique, such as radioimmunoassay (RIA), chemiluminescent or colorimetric enzyme linked immunosorbent assay (ELISA), immunofluorescence assay (IFA), dot blot, western blot, flow cytometry, Luminex assay, ProQuantum immunoassay, or, preferably, mass-spectrometry.

In context of the present invention, it is to be understood as a "responder" a subject in which the improvement in the outcome of the disease to be treated is caused by the specific treatment. A "non responder" is a subject in which the treatment will not cause any expected improvement, or it may even worse the clinical situation of said subject.

In a preferred embodiment of this aspect, the prognosis is performed prior treating the subject with recombinant Protein C, preferably prior treating with recombinant Activated Protein C.

In another embodiment, the present invention also relates to the *in vitro* use of the levels of peptide of SEQ ID NO 1 and/or SEQ ID NO 2 in one or more biological samples isolated from subjects having sepsis or septic shock as a biomarker for screening for subjects at risk of having disseminated intravascular coagulation (DIC).

In another embodiment, the present invention also relates to the *in vitro* use of the peptide of SEQ ID NO 1 and/or SEQ ID NO 2 in one or more biological samples isolated from subjects in need thereof as a biomarker for prognosticating the response to treatment with recombinant PROC, preferably recombinant APC, in a subject having sepsis or septic shock and disseminated intravascular coagulation (DIC). In a preferred embodiment, this *in vitro use* is performed prior treating the subject with recombinant PROC, preferably recombinant APC.

### Compositions and kits

As described above, APC treatment is being used for different pathological conditions, such as sepsis, septic shock, or stroke. In view of this, a **fifth aspect** of the present invention relates to a composition comprising recombinant Protein C (rPROC), preferably recombinant Activated Protein C (rAPC), for use in the treatment of any pathophysiological condition in which endogenous PROC and endogenous APC levels are decreased bellow their physiological levels, wherein the use comprises the step of administering said composition to the subjects that are classified as responders according to the fourth aspect as defined above or any of its embodiments. Preferably, the pathophysiological conditions to be treated are sepsis, sepsis shock, DIC or stroke.

Also preferably, the fifth aspect relates to a composition comprising recombinant Protein C (rPROC), preferably recombinant Activated Protein C (rAPC), for use in the treatment of sepsis or septic shock wherein the use comprises the step of administering said composition to the subjects that are classified as responders according to the fourth aspect described above or any of its embodiments.

In a **sixth aspect,** the present invention relates to a composition comprising recombinant Protein C (rPROC), preferably recombinant Activated Protein C (rAPC), for use in the treatment of a patient selection characterized by having sepsis or sepsis shock and disseminated intravascular coagulation (DIC).

In a **seventh aspect,** the present invention relates to a kit or device suitable for screening or classifying sepsis or septic shock or DIC in a subject and/or for predicting (prognosticating) the response to treatment with recombinant Protein C (rPROC), preferably recombinant Activated Protein C (rAPC), in a subject, which comprises the peptide of SEQ ID NO 1 and/or SEQ ID NO 2, wherein the peptide(s) is(are) preferably synthesized with at least one or more heavy amino acids and wherein said peptide is preferably label in the c-terminal amino acid residue with a tag.

The kit may also contain instructions indicating how the materials within the kit may be used.

Optionally (additionally), the kit can include cleaving enzymes such as trypsin, chymotrypsin, pepsin, papain pepsin, papain, Staphylococcus aureus (V8) protease, Submaxillaris protease, bromelain, thermolysin, aspartate endopeptidase, and/or ArgC. The kit can also include a chemical reagent such as DTT or IAM, or other chemical reagents for chemical generation of protein derived polypeptides.

Optionally, microwave-assisted digestion could be used during enzymatic digestion, preferably with trypsin, ArgC and/or any combination of proteases which give rise after digestion to the peptides of SEQ ID NO 1 and/or SEQ ID NO 2, to accelerate the formation of peptides (P Muralidhar Reddy, et al. Digestion Completeness of Microwave-Assisted and Conventional Trypsin-Catalyzed Reactions. Journal of the American Society for Mass Spectrometry. Volume 21, Issue 3, March 2010, Pages 421-424). The kit could contain trypsin, ArgC and/or any combination of proteases which give rise after digestion to the peptide of SEQ ID NO 1 and/or SEQ ID NO 2, and special microwave-assisted digestion protocol for SEQ ID NO 1 and/or SEQ ID NO 2 optimal analysis.

An **eighth aspect** of the invention refers to the *in vitro* use of the kit as defined in the seventh aspect of the invention or in any of its embodiments, for screening or classifying subjects at risk of having sepsis, septic shock or DIC or for predicting (prognosticating) the response to treatment with recombinant Protein C (rPROC), preferably recombinant Activated Protein C (rAPC) in a subject.

A **ninth aspect** of the invention refers to a computer program product directly loadable into the internal memory of a digital computer, comprising software code portions for performing the steps of comparing the level or concentration of the PROC, preferably APC, from the one or more biological samples of the subject in need thereof with a reference value, and providing a risk of having sepsis, septic shock or DIC or a prediction of being responder/non responder to recombinant PROC or APC treatment, when said product is run on a computer.

The following examples merely illustrate the present invention but do not limit the same.

### EXAMPLES

### EXAMPLE 1: Methods

### Sample size

A total of 157 plasma samples were distributed in 4 groups as follows: 17 samples of reference population controls, 17 ICU controls without any related septic process, 24 cases of sepsis and 99 cases of septic shock. All samples were obtained from reference population collection and sepsis collection from the INCLIVA's Biobank.

### Sample preparation

For the preparation of samples and digestion with trypsin, different protocols were tested. Given the reduced time necessary for the analysis of sera with sepsis, we have optimized an analysis protocol that includes reduction with DTT and microwave digestion with trypsin. The optimized method is described below.

Plasma samples were centrifuged 15min at 15,000g for delipidation. 2µL of each plasma sample was diluted in 8 µL H2O. 2 µL of each 1/5 diluted plasma (aprox. 25 µg) is mixed in 18 µL of 50mM BA together with 25 fmol of Heavy Mix peptides in a 20µL final volume. Cysteine residues were reduced by 2 mM DTT (DL-Dithiothreitol) in 50 mM ABC at 300 W microwave for 3 min. Sulfhydryl groups were alkylated with 5 mM IAM (iodoacetamide) in 50 mM ABC in the dark at r.t. for 20 min. Sample was subjected to trypsin digestion with 5µg (25µg/µl) of sequencing grade-modified trypsin (Promega) in 50 mM ABC at 300 W microwave for 4 min. The reaction was stopped with TFA (trifluoroacetic acid) at a final concentration of 1%.

Time for sample preparation: 45 minutes

### Mass spectrometry analysis

The MRM experiments were performed in a 5500 QTRAP hybrid triple quadrupole/linear ion trap mass spectrometer (SCIEX), equipped with a Micro M3 MicroLC chromatographic system. 10 µL of triptic digest (about 9 µg of protein and 25 fmol of each Spike-In peptide) was injected into a Trap column (10X0.3mm Trap Cartridge Chromxp C18CL 5um, ABSCIEX) and was then loaded and separated onto an analytical column (ChromXP C18, 120 Å, 3 µm, 150x0.3mm). Elution was carried out with a linear gradient of 0 to 35% B in A for 30min. (A: 0.1% FA; B: ACN, 0.1% FA) at a flow rate of 5µl/min.

The 5500 QTRAP was operated in MRM mode. MRM data were acquired in the positive mode with a spray voltage of 5500 V, curtain gas: 25 psi, ion source gas: 25psi, entrance potential (EP):10 and exit potential (EXP): 16. Collision energy (CE) and Declustering Potential (DP) were optimized previously by each transition.

A data analysis was done and the area ratios (light/heavy) for all transitions were calculated using the Skyline 4.2.1.19058 software (MacCoss lab) and light concentration estimated as fmol/µL of initial serum. The heavy peptides used for the analysis were synthetized by CNB (Centro Nacional de Biotecnología-CSIC).

Time for sample analysis: 30 minutes.

The peptides used for the quantification of each protein are the following:

| **Table 1. Peptides used** | |
|---|---|
| **Protein** | **Peptide** |
| Histone H2B | LLLPGELAK (SEQ ID NO.: 3) |
| Histone H3 | STELLIR (SEQ ID NO.: 4) |
| Activated C Protein | LGEYDLR (SEQ ID NO.: 1) |
| | TFVLNFIK (SEQ ID NO.: 2) |

NOTE: in all samples were measured simultaneously Histones (H2B, H3 and H4) and human Activated C Protein.

### Linearity assessment for Spiked-in peptides

First, commercial light peptides for APC containing the sequences SEQ ID NO 1 (LGEYDLR) and SEQ ID NO 2 (TFVLNFIK) were trypsinized and analyzed using LC-MS/MS_DDA in a 5600 triple TOF (SCIEX, Framingham, MA, USA) to identify those unequivocal peptides with good signal. MRM parameters were optimized in a 5500 QTRAP instrument (SCIEX) by the MRM-PILOT software (SCUEX), determining the declustering potential (DP) for every peptide and dwell time (DT) and collision energy for each transition (CE). Afterwards, peptides with high number of detectable transitions, high sensitivity and high linear dinamic range were chosen to perform MRM-MS experiments. Selected peptide sequences (SEQ ID NO 1 (LGEYDLR) and SEQ ID NO 2 (TFVLNFIK)) were prepared as SpikeTidesTM_TQ (heavy-isotope labeled; Arg: 13C6, 15N4; Lys: Arg: 13C6, 15N2) (JPT Peptide Technologies, Berlin, Germany) for absolute quantification.

### ROC curves

The Receiver operating characteristic (ROC) curve were constructed and analyzed in order to obtain: the area under curve (AUC), cut off value, sensitivity and specificity for the APC biomarker for the diagnosis of septic processes and for the identification of DIC in septic shock cases. AUCs above 0.5 (p<0.05) were considered statistically significant. All the analysis were performed with GraPhad Prism v5.

### Statistical analysis

Statistical parameters (mean, standard deviation, minimum, maximum) were calculated for the APC protein in each group of study. For the comparison between groups, first normality tests (Kolmogorov-Smirnov) were performed for the APC variable among groups. Subsequently comparison between groups were performed by using the Mann Whitney U test (for two groups) and the Kruskal-Wallis test with Dunn's post hoc test for comparison between 3 or more groups.

In order to evaluate the correlation between APC values and other clinical and biochemical variables we calculated the Spearman's rank correlation coefficient.

Differences and associations were considered statistically significant when p value of the test were smaller than 0.05. All the analysis were performed with GraPhad Prism v5.

### EXAMPLE 2: Mass spectrometry linearity for the selected peptides of activated protein C.

Initial exploratory studies using the sequence and bioinformatic trypsinization of whole sequence of APC provided several peptide candidates. However, it was finally decided to use the two peptides LGEYDLR and TFVLNFIK. These two peptides (marked below in gray) were generated by peptidecutter-ExPasy [https://web.expasy.org/cgibin/peptide cutter/peptidecutter.pl] using trypsin and generating the cleavage between positions 54 and 60 for LGYDLR (SEQ ID NO.:1) and 156-164 for TFVLNFIK (SEQ ID NO.:2).

### Protein primary sequences of APC

Chain C (SEQ ID NO.:5) (https://www.ncbi.nlm.nih.gov/protein/1AUT C) 
1 lidgkmtrrg dspwqvvlld skkklacgav lihpswvlta ahcmdeskkl lvrlgeydlr
61 rwekweldld ikevfvhpny sksttdndia llhlaqpatl sqtivpiclp dsglaereln
121 qagqetlvtg wgyhssreke akrnrtfvln fikipvvphn ecsevmsnmv senmlcagil
181 gdrqdacegd sggpmvasfh gtwflvglvs wgegcgllhn ygvytkvsry ldwihghird
241 keapqkswap
Chain L (SEQ ID NO.:6) (https://www.ncbi.nlm.nih.gov/protein/1AUT_L)

1 skhvdgdqcl vlplehpcas lccghgtcix gigsfscdcr sgwegrfcqr evsflncsld
61 nggcthycle evgwrrcsca pgyklgddll qchpavkfpc grpwkrmekk rshl

A calibration curve was carried out as explained above. From the results obtained in the calibration curve, LGEYDLR was selected as the preferred peptide for quantification since this peptide showed better linearity than TFVLNFIK (Fig. 1).

It is important to note that both peptides LGEYDLR and TFVLNFIK can be used to measure both endogenous and recombinant forms of PROC and APC (Xigris and 3K3A-APC).

### EXAMPLE 3: Diagnosis of septic processes.

APC is normally distributed in the population and sepsis control groups and is not normally distributed in the septic shock and ICU control group. For all the contrasts to be performed, non-parametric tests will be used that are more robust when the data does not behave normally.

The descriptive statistics for the concentration of APC (ng/mL) in each of the study groups were calculated. In Table 2 are shown the values obtained in cases (septic shock + sepsis) and controls (reference population + ICU non-septic patients).

| **Table 2. Descriptive statistic parameters for APC in cases and controls.** | | | | |
|---|---|---|---|---|
| **Group** | **[APC] ng/mL (IC 95%)** | **Standard Dev.** | **Min.** | **Max.** |
| Cases | 613.70 (590.69-666.65) | 295.60 | 103.57 | 2044.13 |
| Controls | 1431.57 (1231.00-1632.13) | 574.83 | 637.37 | 3734.00 |

Afterwards, we calculated the statistic parameters for the concentration of APC (ng/mL) in each of the subgroups analyzed (Table 3).

| **Table 3. Descriptive statistic parameters for APC in the 4 groups** | | | | |
|---|---|---|---|---|
| **Group** | **[APC] ng/mL (IC 95%)** | **Standard Dev.** | **Min.** | **Max.** |
| Sepsis | 625.98 (528.81-723.15) | 230.12 | 293.76 | 1119.91 |
| Septic Shock | 610.66 (548.41-672.90) | 310.48 | 103.57 | 2044.13 |
| ICU Controls | 1373.72 (1007.59-1739.85) | 712.10 | 637.37 | 3734.00 |
| Reference population | 1489.41 (1273.10-1699.72) | 409.04 | 959.77 | 2165.18 |

Next, the APC concentrations were compared among the different groups of study in order to identify statistically significant differences in the values found.

For the comparison between cases and controls, the Mann Whitney U test is used, which indicates that the differences observed are statistically significant with a value of p <0.0001. For the contrast among the 4 study groups, the Kruskal-Wallis test equivalent to the ANOVA parametric test is used. This test showed a value of p <0.0001 indicating that there were statistically significant differences in PCA levels between the different study groups.

Statistically significant differences were found in the following comparisons:
- Shock vs. ICU controls (p<0.001), Shock vs. reference population (p<0.0001)
- Sepsis vs. ICU controls (p<0.0001), Sepsis vs. reference population (p<0.0001)

From these results it can be deduced that APC can be useful for the diagnosis of septic processes versus controls.

### EXAMPLE 4: APC correlation with other clinical and analytical parameters.

The analysis of the APC and/or PROC levels was conceived as a part of the present invention for the preparation of an *in vitro* diagnostics test for diagnosis and prognosis of sepsis or septic shock. Therefore, a database was prepared in which several clinical and biochemical parameters were also obtained from patients. Next, we proceeded to perform correlation analyses among APC and other clinical variables obtained during the study to find out which other parameters are correlated to APC. The results are shown in Fig. 2 and 3.

In the ICU control group, APC levels showed a significant correlation with functional C protein values (rho Spearman 0.882, p <0.0001). Functional protein C was obtained from the clinical laboratory at the Hospital Universitario Clínico de Valencia by means the use of the kit HemosIL(R) Protein C from Instrumentation Laboratory (A Werfen Company).

In the group of sepsis, APC levels showed a significant correlation with: histone H2B (Spearman's Rho 0.469, p = 0.021), PCT (Spearman's Rho 0.468, p = 0.021), Quick index (Spearman's Rho 0.514, p = 0.010), functional protein C (Spearman's Rho 0.900, p = 0.037).

In the group of cases of septic shock, APC levels show a significant correlation with: platelets (Spearman's Rho 0.374, p = 0.0001), Quick index (Spearman's Rho 0.302 p = 0.003), activated partial thromboplastin time (Spearman's Rho -0.267, p = 0.026), D-dimer (Spearman's Rho - 0.437, p = 0.002) and functional protein C (Spearman's Rho 0.900, p <0.0001).

The reference values for functional protein C were found between 70 and 140%. It is important to highlight that Functional protein C deficiency increases the risk of DIC (disseminated intravascular coagulation), for example, in the presence of sepsis.

We next explored the levels of APC (ng/mL) found in plasma from the different septic and septic shock patients included in our study, and we found that the mean levels of PROC/APC in patients with diagnosed DIC (DIC group) were lower than those patients who did not show this clinical dysfunction (no DIC) (Fig. 4). In order to evaluate the potential of APC in septic processes to predict DIC, clinical variables were reviewed by the clinicians in order to classify septic patients in DIC or not DIC. As shown in Fig. 4, the mean APC levels in samples of patients diagnosed with DIC (average value 468 ng/ml) was statistically reduced (p=0.0014) in comparison with the mean levels of subjects without DIC (average 600 ng/ml). This classification based on the present invention permitted to identify 14 patients with DIC and 85 patients without DIC by using the measurement of APC by means of the methods of the present invention. The corresponding ROC curve was constructed and analyzed (see Example 7).

These findings are very important, because in a study performed by Papageorgiou et al., 2018 [Papageorgiou C, et al. Disseminated Intravascular Coagulation: An Update on Pathogenesis, Diagnosis, and Therapeutic Strategies. Clin Appl Thromb Hemost. 2018;24(9_suppl):8S-28S. doi:10.1177/1076029618806424] demonstrated that treatment with APC reduced 28-day mortality of patients with severe sepsis, including patients retrospectively assigned to a subgroup with sepsis-associated DIC. Interestingly, treatment with APC did not have any positive effects in other patient groups, suggesting that only septic patients with DIC would be benefit from the use of APC treatment (i.e. Xigris or other rhAPC). In this study, it was also found that the APC treatment increased the bleeding risk in patients with sepsis, so it is very important to accurately diagnose sepsis and measure simultaneously the endogenous levels of APC. Even more the use of APC derivatives without capacity of bleeding would be of outmost importance in the treatment of septic patients.

### EXAMPLE 5: ROC curve for the screening of septic processes (sepsis or septic

### shock) vs. controls.

In order to evaluate the screening potential of APC in septic processes versus controls (reference population + ICU controls), the corresponding ROC curve was constructed and analyzed.

| **Table 4. ROC Curve for APC in the diagnosis of septic process** | | | | | |
|---|---|---|---|---|---|
| **Biomarker** | **AUC IC 95%** | **p** | **Cut-off value ng/mL** | **Sensitivity (%)** | **Specificity (%)** |
| **APC** | 0.943 (0.907-0.978) | <0.0001 | 800 | 79.5 | 97.1 |

### [ 1] Simple diagnostic tests

| | |
|---|---|
| Confidence level: | 95,0% |

**Reference test**

| Diagnostic test | Patients | Controls | Total |
|---|---|---|---|
| Positive | 97 | 1 | 98 |
| Negative | 25 | 33 | 58 |
| Total | 122 | 34 | 156 |

| | Value | CI (95%) | |
|---|---|---|---|
| **Sensitivity (%)** | **79,51** | 71,94 | 87,08 |
| **Specificity (%)** | **97,06** | 89,91 | 100,00 |
| Validity index (%) | 83,33 | 77,16 | 89,50 |
| **Positive predictive value (%)** | **98,98** | 96,48 | 100,00 |
| **Negative Predictive value (%)** | **56,90** | 43,29 | 70,50 |
| Prevalence (%) | 78,21 | 71,41 | 85,00 |
| Youden Index | 0,77 | 0,67 | 0,86 |
| **Positive likelihood ratio test** | **27,03** | 3,91 | 186,81 |
| **Negative likelihood ratio test** | **0,21** | 0,15 | 0,30 |

It can thus be observed that, according to the sensitivity and specificity values obtained in our statistical analysis, the test based in the PROC/APC biomarker is reliable. The method of the present invention is able to classify properly the 79.5% of the septic cases (sepsis or septic shock) and the 97.06 % of the controls.

In this regard, the predictive values could be considered acceptable, and reliable for the classification as a case when a positive result of the test is obtained (PPV 98.98).

These conclusions are supported by a positive likelihood ratio much greater than 1, and a negative likelihood ratio far from 1.

### EXAMPLE 6: ROC curve for the screening of septic processes (sepsis or septic shock) vs. ICU controls.

In order to evaluate the diagnostic potential of APC in septic processes versus ICU controls in a tertiary hospital ICU, the corresponding ROC curve was constructed and analyzed.

| **Table 5. ROC curve for APC in the diagnosis of septic process** | | | | | |
|---|---|---|---|---|---|
| **Biomarker** | **AUC IC 95%** | **p** | **Cut-off value ng/mL** | **Sensitivity (%)** | **Specificity (%)** |
| **APC** | 0.920 (0.867-0.974) | <0.0001 | 800 | 79.5 | 94.1 |

### Simple diagnostic tests

| | |
|---|---|
| Confidence level: | 95,0% |

**Reference test**

| Diagnostic test | Patients | Controls | Total |
|---|---|---|---|
| Positive | 97 | 1 | 98 |
| Negative | 25 | 16 | 41 |
| Total | 122 | 17 | 139 |

| | Value | CI (95%) | |
|---|---|---|---|
| **Sensitivity (%)** | **79,51** | 71,94 | 87,08 |
| **specificity (%)** | **94,12** | 79,99 | 100,00 |
| Validity index (%) | 81,29 | 74,45 | 88,14 |
| **Positive predictive value (%)** | **98,98** | 96,48 | 100,00 |
| **Negative predictive value (%)** | **39,02** | 22,87 | 55,18 |
| Prevalence (%) | 87,77 | 81,96 | 93,58 |
| Youden Index | 0,74 | 0,60 | 0,87 |
| **Positive likelihood ratio test** | **13,52** | 2,01 | 90,69 |
| **Negative likelihood ratio test** | **0,22** | 0,15 | 0,31 |

It can thus be observed that according to the sensitivity and specificity values obtained in our statistical analysis, the test based in the PROC/APC biomaker is reliable. The test is able to classify properly the 79.5% of the septic cases and the 94.12 % of the controls.

In this regard, the predictive values could be considered acceptable, and reliable for the classification as a case when a positive result of the test is obtained (PPV 98.98).

These conclusions are supported by a positive likelihood ratio much greater than 1, and a negative likelihood ratio far from 1.

### EXAMPLE 7: ROC curve for the classification of patients at risk of having DIC.

In order to evaluate the potential of APC in septic processes to predict DIC, clinical variables were reviewed by the clinicians in order to classify septic patients in DIC or not DIC. This classification permitted to identify 14 patients with DIC and 85 patients without DIC by using the measurement of APC by MS. The corresponding ROC curve was constructed and analyzed.

| **Table 5. ROC curve for APC in the identification of DIC in septic process** | | | | | |
|---|---|---|---|---|---|
| **Biomarker** | **AUC IC 95%** | **p** | **Cut-off value ng/mL** | **Sensitivity (%)** | **Specificity (%)** |
| **APC** | 0.768 (0.659-0.879) | 0.0013 | 468 | 71.4 | 71.4 |

It can thus be observed that, according to the sensitivity and specificity values obtained in our statistical analysis, the method based in the measurement of the levels of PROC/APC proteins is reliable. The test is able to classify properly the 71.4% of the DIC cases and the 71.4 % of the controls.

These conclusions are supported by a positive likelihood ratio much greater than 1, and a negative likelihood ratio far from 1.

## Claims

1. A mass spectrometry-based method for measuring the levels of Protein C (PROC), preferably Activated Protein C (APC), in a subject in need thereof, the method comprising a step of performing an enzymatic digestion to one or more biological samples isolated from the subject, and a step of measuring by using a mass spectrometer the levels of the peptide of SEQ ID NO 1 (LGEYDLR), wherein the levels of SEQ ID NO 1 (LGEYDLR) correspond to the levels of PROC, preferably APC, present in said one or more biological samples.

2. The mass spectrometry-based method of claim 1, wherein the enzyme used for the enzymatic digestion is trypsin and/or ArgC, or any combination of proteases which give rise after digestion to the LGEYDLR peptide of SEQ ID NO 1.

3. The mass spectrometry-based method of claims 1 and 2, wherein the measurement of the peptide of SEQ ID NO 1 (LGEYDLR) is carried out by spiking-in a labelled proteotypic peptide of SEQ ID NO 1 (LGEYDLR).

4. The mass spectrometry-based method of any of claims 1 to 3, wherein the spike-in labelled proteotypic peptide of SEQ ID NO 1 (LGEYDLR) is synthesized with at least one or more heavy amino acids and wherein said peptide is preferably labelled in the C-terminal amino acid residue with a tag.

5. The mass spectrometry-based method of claims 1 to 4, wherein the method further comprises a step of reducing the one or more biological samples with a reducing agent, and a step of alkylating the one or more biological sample with an alkylating agent, wherein both the reduction and the alkylation steps are performed before the step of the enzymatic digestion.

6. The mass spectrometry-based method of any of claims 1 to 5, wherein the one or more biological samples are selected from the group consisting of blood, serum, plasma, saliva, urine or cerebrospinal fluid.

7. A mass spectrometry-based method for screening or classifying subjects at risk of having sepsis or septic shock, the method comprising the steps of:
i) measuring the levels of Protein C (PROC), preferably Activated protein C (APC), by carrying out the method of any of claims 1 to 6, and
ii) comparing the levels obtained in step i) with a reference value,
wherein a statistically significant variation of the measurement of step i) with the reference value of ii) is indicative that the subject is at risk of having sepsis or septic shock.

8. The mass spectrometry-based method according of claim 7, wherein the reference value of step ii) is obtained from a healthy subject or a population of healthy subjects and wherein a statistically significant reduction in the levels of Protein C, preferably Activated Protein C, is indicative that the subject is at risk of having sepsis or septic shock.

9. The mass spectrometry-based method according to any of claims 7 and 8, wherein the method further comprises a step of clinical confirmation that the patient has sepsis or septic shock.

10. A mass spectrometry-based method for screening or classifying subjects at risk of having disseminated intravascular coagulation (DIC), wherein the subjects have been diagnosed with sepsis or septic shock, the method comprising the steps of:
i) measuring the levels of Protein C (PROC), preferably of Activated protein C (APC), by carrying out the methods of any of claims 1 to 6, and
ii) comparing the levels obtained in step i) with a reference value,
wherein a statistically significant variation of the measurement of step i) with the reference value of ii) is indicative that the subject is at risk of having DIC.

11. The mass spectrometry-based method according to any of claim 10, wherein the reference value of step ii) is obtained from a subject or a population of subjects having sepsis or septic shock but not having disseminated intravascular coagulation (DIC) and wherein a statistically significant reduction in the levels of Protein C (PROC), preferably Activated Protein C (APC), is indicative that the subject is at risk of having DIC.

12. The mass spectrometry-based method according to any of claims 10 and 11, wherein the method further comprises a step of clinical confirmation that the patient has disseminated intravascular coagulation.

13. A mass spectrometry-based method for prognosticating the response to treatment with recombinant Protein C (PROC), preferably recombinant Activated Protein C (APC), in a subject in need thereof, the method comprising the steps of:
i) screening if the subject is at risk of having disseminated intravascular coagulation (DIC) by carrying out each of the steps of the method of any of claims 10 to 13,
ii) classifying the subject as responder to said treatment if the subject is identified at risk or diagnosed with DIC and as non-responder to said treatment if the subject is not identified at risk nor diagnosed with DIC.

14. The method of claim 13, wherein the prognosis is performed prior treating the subject with recombinant Protein C (rPROC), preferably recombinant Activated Protein C.

15. A composition comprising recombinant Protein C (rPROC), preferably recombinant Activated Protein C (rAPC), for use in the treatment of sepsis, septic shock or stroke wherein the use comprises the step of administering said composition to the subjects that are classified as responders according to any of claims 13 to 14.

16. An *in vitro* use of the levels of peptide of SEQ ID NO 1 (LGEYDLR) in one or more biological samples isolated from subjects in need thereof as a biomarker for screening for subjects at risk of having sepsis or sepsis shock.

17. An *in vitro* use of the levels of peptide of SEQ ID NO 1 (LGEYDLR) in one or more biological samples isolated from subjects having sepsis or septic shock as a biomarker for screening for subjects at risk of having disseminated intravascular coagulation.

18. An *in vitro* use of the levels of peptide of SEQ ID NO 1 (LGEYDLR) in one or more biological samples isolated from subjects in need thereof as a biomarker for prognosticating the response to treatment with recombinant PROC, preferably recombinant APC, in a subject having sepsis or septic shock and disseminated intravascular coagulation (DIC).


SEQUENCE LISTING

<110> Consorcio Centro de Investigación Biomédica en Red, M.P.
      Universitat de València
      Instituto de Investigación Sanitaria INCLIVA

<120> METHODS FOR THE MEASUREMENT OF PROTEIN C AND ACTIVATED PROTEIN C

<130> 905 781

<160> 6

<170> BiSSAP 1.3.6

<210> 1
<211> 7
<212> PRT
<213> Artificial Sequence


<220>
<223> Activated C Protein

<400> 1
Leu Gly Glu Tyr Asp Leu Arg
1               5

<210> 2
<211> 8
<212> PRT
<213> Artificial Sequence


<220>
<223> Activated C Protein

<400> 2
Thr Phe Val Leu Asn Phe Ile Lys
1               5

<210> 3
<211> 9
<212> PRT
<213> Artificial Sequence


<220>
<223> Histone H2B

<400> 3
Leu Leu Leu Pro Gly Glu Leu Ala Lys
1               5

<210> 4
<211> 7
<212> PRT
<213> Artificial Sequence


<220>
<223> Histone H3

<400> 4

Ser Thr Glu Leu Leu Ile Arg
1               5

<210> 5
<211> 250
<212> PRT
<213> Homo sapiens



<220>
<223> Protein primary sequences of APC Chain C

<400> 5
Leu Ile Asp Gly Lys Met Thr Arg Arg Gly Asp Ser Pro Trp Gln Val
1               5                   10                  15
Val Leu Leu Asp Ser Lys Lys Lys Leu Ala Cys Gly Ala Val Leu Ile
            20                  25                  30
His Pro Ser Trp Val Leu Thr Ala Ala His Cys Met Asp Glu Ser Lys
        35                  40                  45
Lys Leu Leu Val Arg Leu Gly Glu Tyr Asp Leu Arg Arg Trp Glu Lys
    50                  55                  60
Trp Glu Leu Asp Leu Asp Ile Lys Glu Val Phe Val His Pro Asn Tyr
65                  70                  75                  80
Ser Lys Ser Thr Thr Asp Asn Asp Ile Ala Leu Leu His Leu Ala Gln
                85                  90                  95
Pro Ala Thr Leu Ser Gln Thr Ile Val Pro Ile Cys Leu Pro Asp Ser
            100                 105                 110
Gly Leu Ala Glu Arg Glu Leu Asn Gln Ala Gly Gln Glu Thr Leu Val
        115                 120                 125
Thr Gly Trp Gly Tyr His Ser Ser Arg Glu Lys Glu Ala Lys Arg Asn
    130                 135                 140
Arg Thr Phe Val Leu Asn Phe Ile Lys Ile Pro Val Val Pro His Asn
145                 150                 155                 160
Glu Cys Ser Glu Val Met Ser Asn Met Val Ser Glu Asn Met Leu Cys
                165                 170                 175
Ala Gly Ile Leu Gly Asp Arg Gln Asp Ala Cys Glu Gly Asp Ser Gly
            180                 185                 190
Gly Pro Met Val Ala Ser Phe His Gly Thr Trp Phe Leu Val Gly Leu
        195                 200                 205
Val Ser Trp Gly Glu Gly Cys Gly Leu Leu His Asn Tyr Gly Val Tyr
    210                 215                 220
Thr Lys Val Ser Arg Tyr Leu Asp Trp Ile His Gly His Ile Arg Asp
225                 230                 235                 240
Lys Glu Ala Pro Gln Lys Ser Trp Ala Pro
                245                 250

<210> 6
<211> 114
<212> PRT
<213> Homo sapiens



<220>
<223> Protein primary sequences of APC Chain L

<220>
<221> VARIANT
<222> 30
<223> x = non_std_residue="BHD

<400> 6
Ser Lys His Val Asp Gly Asp Gln Cys Leu Val Leu Pro Leu Glu His
1               5                   10                  15

Pro Cys Ala Ser Leu Cys Cys Gly His Gly Thr Cys Ile Xaa Gly Ile
            20                  25                  30
Gly Ser Phe Ser Cys Asp Cys Arg Ser Gly Trp Glu Gly Arg Phe Cys
        35                  40                  45
Gln Arg Glu Val Ser Phe Leu Asn Cys Ser Leu Asp Asn Gly Gly Cys
    50                  55                  60
Thr His Tyr Cys Leu Glu Glu Val Gly Trp Arg Arg Cys Ser Cys Ala
65                  70                  75                  80
Pro Gly Tyr Lys Leu Gly Asp Asp Leu Leu Gln Cys His Pro Ala Val
                85                  90                  95
Lys Phe Pro Cys Gly Arg Pro Trp Lys Arg Met Glu Lys Lys Arg Ser
            100                 105                 110
His Leu